# EUROPÄISCHE PATENTSCHRIFT

(11) **EP 1 761 523 B1**
(45) Veröffentlichungstag und Bekanntmachung des Hinweises auf die Patenterteilung: **04.11.2015**
(21) Anmeldenummer: 05761654.2
(22) Anmeldetag: 15.06.2005
(51) Int. Cl.: C07D 403/14, D21H 21/30

(54) **VERWENDUNG VON TRIAZINYLFLAVONATAUFHELLERN**
USE OF TRIAZINYL FLAVONATE BRIGHTENERS
APPLICATION D' AZURANTS OPTIQUES A BASE DE TRIAZINYL-FLAVONATES

(30) Priorität: 28.06.2004 DE 102004031101
(43) Veröffentlichungstag der Anmeldung: 14.03.2007
(73) Patentinhaber: Blankophor GmbH & Co. KG, 49577 Ankum (DE)
(72) Erfinder: GIESECKE, Heinz, 51519 Odenthal (DE); GOTTSCHALK, Reiner, 51065 Köln (DE); HUNKE. Bernhard, 53783 Eitorf (DE); PFUETZENREUTER, Dirk, 51399 Burscheid (DE)
(74) Vertreter: Lang, Johannes
(86) Internationale Anmeldenummer: PCT/EP2005/006421
(87) Internationale Veröffentlichungsnummer: WO 2006/000327

(56) Entgegenhaltungen:
- EP-A- 0 485 329
- EP-A- 1 355 004
- DE-A1- 1 949 137
- DATABASE HCAPLUS [Online] ACS; XP002356446 gefunden im STN Database accession no. 63:69161 & PALYI, GYULA: MAGYAR KEMIKUSOK LAPJA, Bd. 71, 1965, Seiten 120-6,
- DATABASE HCAPLUS [Online] ACS; XP002356447 gefunden im STN Database accession no. 77:90029 & YAMADA, KIMIHO ET AL.: YUKI GOSEI KAGAKU KYOKAISHI, Bd. 29, 1971, Seiten 1121-8,

## Beschreibung

Die Erfindung betrifft die Verwendung von Triazinylflavonataufhellern als Aufheller für den Papierstrich sowie diese enthaltende Streichmassen.

Bei der Herstellung gestrichener Papiere ist der Zusatz optischer Aufheller zur Streichfarbe üblich, so dass sich der optische Aufheller beim fertig gestrichenen Papier auch in der auf das Papier aufgebrachten Pigmentschicht befindet. Gestrichene Papiere sind besonders zur Anfertigung hochwertiger Drucke geeignet. Neben guten Bedruckbarkeitseigenschaften wird ihre Qualität daher hauptsächlich nach optischen Eigenschaften wie Glanz, Glätte und Weiße beurteilt. Es besteht ein fortgesetzter Trend zu gestrichenen Papieren mit hohen Weißgraden und daher der Wunsch nach möglichst effektiven optischen Aufhellern als Streichfarbenkomponenten.

Triazinylflavonataufheller werden weltweit in großem Umfang zum Weißtönen von Papier und Textilien in der Papier-, Textil- und Waschmittelindustrie eingesetzt. Im Allgemeinen ist diese Aufhellerklasse chemisch aufgebaut aus einem 4,4'-Diamino-stilben-2,2'-disulfonsäure-Mittelstück und zwei Triazinresten, die zusätzlich 4 gegebenenfalls weiter substituierte Amin- und/oder Alkoxy- bzw. Aryloxyreste enthalten. Triazinylflavonataufheller dieser Struktur mit nur einem 4,4'-Diamino-stilben-2,2'-disulfonsäurekern, nachfolgend als (bezüglich des Mittelstücks) einkernige Triazinylflavonataufheller bezeichnet, sind geeignet, einen großen Umfang an gewünschten Aufhellungseffekten in den genannten Anwendungsgebieten und insbesondere auch im Papierstrich zur Verfügung zu stellen.

Entsprechende einkernige Triazinylflavonataufheller für den Papierstrich sind seit langem bekannt beispielsweise aus EP-A- 1 355 004 und sind noch immer hinsichtlich ihres Weißgrades verbesserungsfähig.

GB 732 139 A offenbart die Herstellung von Triazinyl-verbrückten Diaminostilbendisulfonsäureverbindungen, die als Zusatz zu Zellstoff bei der Papierherstellung oder zu Waschmitteln verwendet werden können. DATABASE HCAPLUS, ACS, accession no. 63:69161/DN (XP002356446) & PALYI, GYULA:MAGYAR KEMIKUSOK LAPJA, Bd. 71, 1965, Seiten 120-6 offenbaren spezifische Aufheller für polarographische Adsorption. DATABASE HCAPLUS, ACS, accession no. 77:90029/DN (XP002356447); & YAMADA, KIMIHO ET AL.:YUKI GOSEI KAGAKU KYOKAISHI, Bd. 29, 1971, Seiten 1121-8 offenbaren blaue Farbstoffe, die spezifische Aufheller enthalten. EP 0 485 329 A betrifft Triphendioxazinverbindungen einer bestimmten Formel, die zum Färben oder Bedrucken von textilen Fasermaterialien verwendet werden. DE 19 49 137 A1 offenbart oligomere optische Aufhellerverbindungen einer bestimmten Formel zur Verwendung in Waschmitteln.

Auf Grund des oben erwähnten fortgesetzten Trends zu gestrichenen Papieren mit hohen Weißgraden und des daher bestehenden Wunsches nach möglichst effektiven optischen Aufhellern besteht infolgedessen Interesse daran, optische Aufheller mit gegenüber den bekannten einkernigen Triazinylflavonataufhellern erhöhter Effizienz zur Verfügung zu stellen.

Überraschenderweise wurden nun Verbindungen der Formel Ia gefunden, die erfindungsgemäß als Aufheller in Papierstreichmassen verwendet werden und die im obigen Sinn als zweikernige Triazinylflavonataufheller bezeichnet werden können, 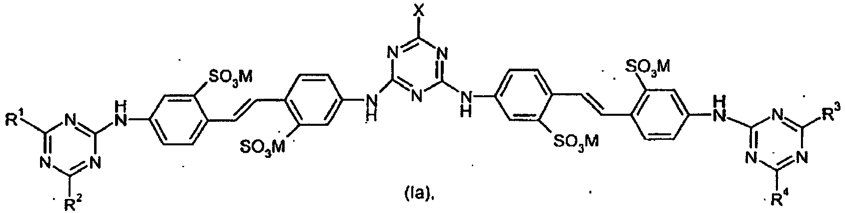 worin
R¹ und R³ unabhängig voneinander für einen Rest der Formel 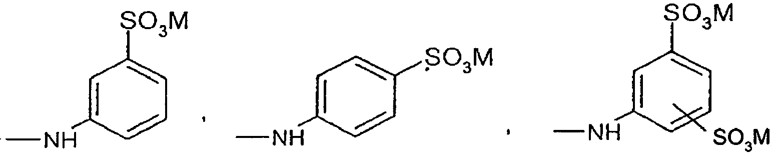 steht,
- R² und R⁴: unabhängig voneinander für -NHCH₂-CH₂-OH, -N(CH₂CH₂OH)₂, 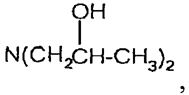 Anilino oder Morpholino steht und
- X: für -OH oder für -NHCH₂CH₂OH, -N(CH₂CH₂OH)₂, 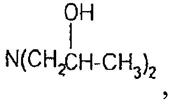 Anilino oder Morpholino steht und
- M: für Wasserstoff, ein Äquivalent eines ein- oder zweiwertigen Metallions, insbesondere aus der Gruppe der Alkali- oder Erdalkalimetalle oder ein gegebenenfalls organisch substituiertes Ammoniumion steht.

Bevorzugte Verbindungen der Formel Ia sind solche, in denen wenigstens einer der Reste R¹ oder R² die gleiche Bedeutung wie wenigstens einer der Reste R³ oder R⁴ hat. Besonders bevorzugt ist R¹= R³ und R² = R⁴. Ebenfalls besonders bevorzugt sind die Reste R¹ bis R⁴ identisch.

Bevorzugt steht M für Wasserstoff oder Alkali- oder Erdalkalimetalle, insbesondere für Lithium, Natrium, Kalium oder ein Äquivalent des Magnesiums oder Calciums, sowie besonders bevorzugt Ammonium und hydroxyalkylsubstituiertes, insbesondere hydroxysubstituiertes C₁-C₄-Alkylammonium.

Ein weiterer Gegenstand der vorliegenden Erfindung ist die Verwendung der Verbindungen der Formel Ia, die dadurch gekennzeichnet ist, dass man Verbindungen der Formel Ia herstellt, indem man eine Verbindung der Formel II 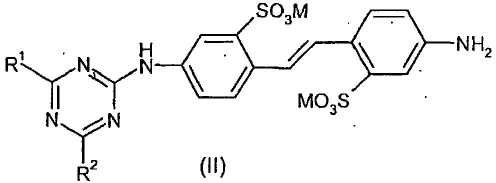 worin
R¹, R² und M die oben angegebene Bedeutung haben, mit einer Verbindung der Formel III

Y-B¹-Z (III)

worin
- Y und Z: unabhängig voneinander für Abgangsgruppen stehen, die durch die freie Aminogruppe der Verbindungen der Formel II ersetzt werden können und
- B¹: für einen Rest der Formel 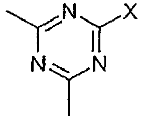 steht und X die obige Bedeutung hat, zu einer Verbindung der Formel IIIa umsetzt 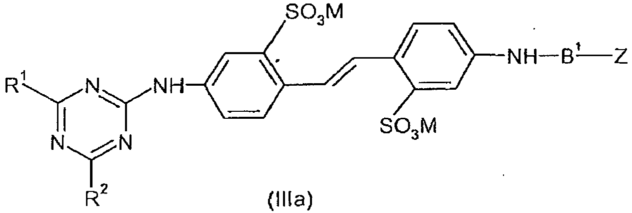 worin
- R¹, R², B¹ und Z: die oben genannte Bedeutung haben,
und die Verbindung IIIa weiter mit einer Verbindung der Formel IV umsetzt, 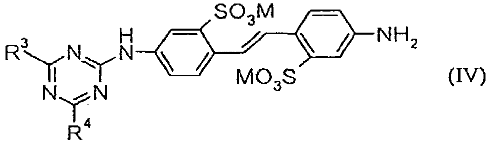 in der R³, R⁴ und M die oben beschriebene Bedeutung haben.

Besonders bevorzugt sind solche Brückenglieder B¹, wobei X insbesondere mit wenigstens einem der Reste R¹ bis R⁴ identisch ist oder für Hydroxy steht.

Unter Abgangsgruppen Y und Z in Formel III sind vorzugsweise Molekülteile zu verstehen, die untereinander verschieden oder auch gleich sind und die bei einer Kondensationsreaktion einer Verbindung der Formel II bzw. IV mit einer Verbindung der Formel III als Verbindungen Y-H bzw. Z-H abgespalten werden. Vorzugsweise ist die Abspaltung der 2. Abgangsgruppe weniger leicht als die der ersten, so dass eine möglichst selektive 1:1-Umsetzung der Verbindung der Formel II mit der Verbindung der Formel III in der Weise erfolgen kann, dass im entstehenden Kondensationsprodukt im Wesentlichen nur eine der Gruppen Y oder Z enthalten bleibt, wobei dies besonders dann wichtig ist, wenn unsymmetrische Verbindungen der Formel Ia hergestellt werden sollen, d.h. wenigstens R¹ ≠ R³ und/oder R² ≠ R⁴ ist.

Bevorzugt stehen Y bzw. Z unabhängig voneinander für Halogen, insbesondere Fluor, Chlor oder Brom, insbesondere Chlor, Alkoxy, insbesondere C₁-C₄-Alkoxy oder Aryloxy, insbesondere gegebenenfalls substituiertes Phenoxy.

Als bevorzugte Verbindungen der Formel III kommen beispielsweise Chlorkohlensäureester, insbesondere deren C₁-C₄-Alkylester oder 2,4,6-Trichlortriazin in Frage.

Bevorzugt erfolgt die Umsetzung von II mit III bei einer Temperatur von 5 bis 50°C. Das bevorzugte molare Verhältnis von II zu III beträgt 0,85 bis 1,15.

Die Umsetzung des Reaktionsproduktes von II und III - also IIIa - mit Verbindungen der Formel IV erfolgt vorzugsweise bei einer Temperatur von 20 bis 100°C. Das molare Verhältnis von II zu IV beträgt vorzugsweise 0,95 bis 1,05.

Als Reaktionsmedium zur Umsetzung der Verbindungen der Formeln II-IV kommen vorzugsweise Wasser oder mit Wasser vollständig oder teilweise mischbare organische Lösungsmittel oder Gemische daraus in Betracht, z.B. Aceton, Methylethylketon, Methylethylketon-Wasser-Mischungen usw. Dabei können die Reaktanden entweder gelöst oder auch als Suspension oder Emulsion oder Mischformen davon vorliegen.

Besonders bevorzugt ist ein wässriges Reaktionsmedium, das ggf. weitere Komponenten wie z.B. anorganische Salze, Emulgatoren, etc. enthalten kann.

Der pH-Wert der Reaktionsmischung wird für die einzelnen Reaktionsstufen bevorzugt so gewählt, dass die jeweils durchgeführte Stufe möglichst selektiv und mit hoher Geschwindigkeit ablaufen kann.

Für den Fall, dass die Substanz der Formel III außer Y und Z noch eine weitere Abgangsgruppe enthält, kann diese vorzugsweise durch darauf folgende Umsetzung mit einem Alkohol R⁵OH oder einem Amin (R⁶, R⁷)NH, wobei R⁵, R⁶ und R⁷ die unten angegebene Bedeutung haben, in das Endprodukt der Formel Ia umgewandelt werden.

In einer bevorzugten Ausführungsform der Erfindung wird das Verfahren unter Verwendung identischer Verbindungen der Formel II und der Formel IV durchgeführt.

Die Verbindungen der Formel II bzw. IV lassen sich nach an sich bekannten Methoden herstellen, indem man beispielsweise Cyanurchlorid, vorzugsweise jeweils im molaren Verhältnis von ca. 1:1; in beliebiger Reihenfolge mit einer Verbindung der Formel V 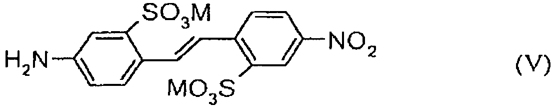 worin
- M: die oben beschriebene Bedeutung hat,
und mit Verbindungen der Formeln R¹H und R²H bzw. R³H und R⁴H, wobei R¹-R⁴ die oben angegebene Bedeutung haben, umsetzt und im Anschluss daran die Nitrogruppe der entstandenen Verbindungen der Formel VI 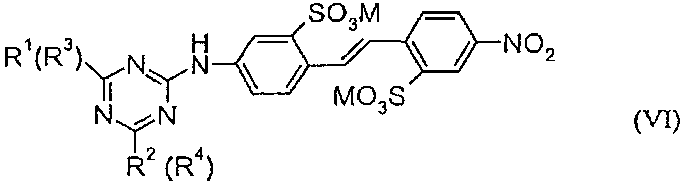 in an sich bekannter Weise zur Aminogruppe reduziert.

Die Aufheller der Formel Ia haben insbesondere bei Anwendung im pigmentierten Strich auf Papier vorzugsweise in Kombination mit anderen Flavonataufhellern ein deutlich verbessertes Weißaufbauverhalten gegenüber den nicht verdoppelten Aufhellern. Somit kann der gewünschte Aufhelleffekt mit einer geringeren Menge an Weißtöner erreicht werden.

Die Erfindung betrifft weiterhin die Verwendung der Aufheller in Zubereitungen enthaltend wenigstens einen Aufheller der Formel Ia.

Die genannten Zubereitungen können zusätzlich zu den erfindungsgemäß verwendeten Triazinylflavonataufhellern weitere Substanzen enthalten, beispielsweise Wasser, Carriersubstanzen, Salze und Stellmittel. Sie können außerdem auch größere Mengen bereits bekannter Aufheller aus der Gruppe der Triazinylflavonataufheller enthalten, wobei solche, die nur einen 4,4'-Diamino-stilben-2,2'-disulfonsäurerest enthalten, bevorzugt sind.

Im Allgemeinen enthalten die Zubereitungen die Triazinylflavonataufheller der Formel Ia, bezogen auf Gesamt-triazinylflavonataufheller, in einer Menge von 1 bis 20 Flächenprozente. Dieser prozentuale Flächenanteil wird gemessen in einem Hochdruckflüssigkeitschromatogramm bei einer Wellenlänge von 350 nm an einer wässrigen Messlösung der Zubereitung, wobei die für 100 ml Messlösung einzuwiegende, in Gramm ausgedrückte Menge der Zubereitung, multipliziert mit ihrem E1/1-Wert, 50 betragen soll. Wässrige Aufhellerzubereitungen werden üblicherweise durch den sogenannten E1/1-Wert charakterisiert. Dazu wird die Extinktion einer stark verdünnten Lösung der Zubereitung nach den üblichen und dem Fachmann bekannten Methoden der UV/Vis-Spektroskopie in einer 1cm-Küvette bei einer bestimmten Wellenlänge bestimmt. Diese Wellenlänge entspricht dem langwelligen Absorptionsmaximum des jeweiligen Aufhellermoleküls. Bei Flavonataufhellern beträgt sie ca. 350 nm. Der E1/1-Wert entspricht dann dem fiktiven, auf eine 1 %ige Lösung der zu bestimmenden Probe hochgerechneten Extinktionswert.

Bevorzugte Messbedingungen für das Hochdruckflüssigkeitschromatogramm sind: Trennung mit einer reverse-phase (RP)-Säule und wässrigen Eluenten (beispielsweise gepuffert, mit Acetonitril und Ionenpaarreagenz).

Bevorzugte Zubereitungen enthalten die Triazinylflavonataufheller der Formel Ia, bezogen auf Gesamtaufheller, in einer Menge von 1 bis 15 %, insbesondere 1 bis 10 Flächenprozente, 2 bis 10 Flächenprozente, ganz besonders bevorzugt 2,5 bis 10 Flächenprozente, insbesondere 3,0 bis 10 Flächenprozente wie oben bestimmt. Weitere bevorzugte Untergrenzen sind 3,5; 4,0; 4,5 und 5,0 Flächenprozente.

Des Weiteren können die Zubereitungen als wässrige Präparationen, insbesondere als Lösungen vorliegen, aber auch feste Formen aufweisen und z.B. als Pulver oder Granulat vorliegen.

Bevorzugte Zubereitungen sind wässrig und enthalten
- 5 bis 50 Gew.-%: Aufheller, wobei wenigstens einer der Aufheller der Verbindung der Formel Ia entspricht,
- 0 bis 60 Gew.-% Carrier.:

Als bevorzugter mit zu verwendender Aufheller in der Zubereitung wird ein Aufheller der Formel VII eingesetzt 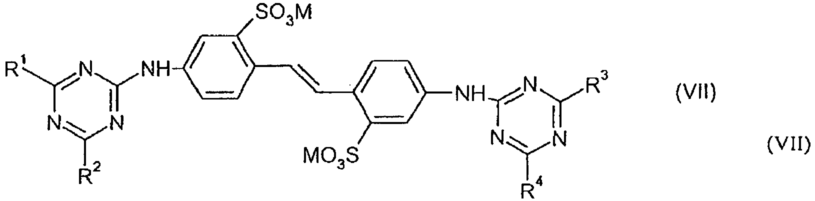 worin
- R¹ bis R⁴ und M: unabhängig von der Bedeutung in Formel Ia die Bedeutung besitzen, dass
- R¹, R², R³ und R⁴: jeweils unabhängig voneinander für OR⁵ oder NR⁶R⁷ stehen, wobei
- R⁵, R⁶ und R⁷: jeweils unabhängig voneinander für Wasserstoff, substituiertes oder unsubstituiertes Alkyl, insbesondere C₁-C₄-Alkyl oder substituiertes oder unsubstituiertes Aryl, insbesondere C₆-C₁₀-Aryl stehen, wobei
- R⁶, R⁷: zusammen mit dem N-Atom, an das sie gebunden sind und gegebenenfalls weiteren Heteroatomen auch einen aliphatischen oder aromatischen Ring bilden können, und
- M: die oben angegebene Bedeutung besitzt.

Bevorzugte Verbindungen VII sind solche, in denen wenigstens einer der Reste R¹ oder R² die gleiche Bedeutung wie wenigstens einer der Reste R³ oder R⁴ hat.

Bevorzugte Reste R¹, R², R³ und R⁴ in der Verbindung VII sind die folgenden: Phenoxy, mono- oder disulfoniertes Phenoxy, Phenylamino, mono- oder disulfoniertes Phenylamino, Phenylamino substituiert durch C₁-C₃-Alkyl, Cyano, Halogen, insbesondere Cl oder Br, COOR, CONH-R, NH-COR, SO₂NH-R, OR, weiterhin die Reste Morpholino, Piperidino, Pyrrolidino, -OC₁-C₄-Alkyl,-NH-(C₁-C₄-Alkyl)-, -N (C₁-C₄-Alkyl)₂, -NH(C₂-C₄-Alkylen)-OR, N[(C₂-C₄-Alkylen)-OR]₂, NH(C₂-C₄-Hydroxyalkyl), -N(C₂-C₄-Hydroxyalkyl)₂, -NH(C₂-C₄-Alkylen-O-C₂-C₄-Alkylen-OR), eine Aminosäure bzw. Aminosäuresalz oder ein Aminosäureamid, von deren basischer Aminogruppe ein Wasserstoffatom entfernt wurde, -N(CH₃)(CH₂CH₂OH), -NH₂, -OCH₂CH₂SO₃M, -NH-CH₂CH₂SO₃M, -N(CH₂CH₂SO₃M)₂ oder -N(CH₂CH₂OH)CH₂CH₂CONH₂, wobei R = H oder C₁-C₃-Alkyl bedeutet und M die oben angegebene Bedeutung hat.

Von diesen sind besonders bevorzugt die Reste -NH₂, NH-CH₃, -NH-C₂H₅, -N(CH₃)₂, -N(C₂H₅)₂,-NH-C₂-C₄-Hydroxyalkyl, insbesondere NH-CH₂-CH₂OH, -N(C₂-C₄-Hydroxyalkyl)₂, -NH-CH₂CH₂SO₃M, -NH-CH₂-CH₂-O-CH₂-CH₂-OH, -OCH₃, -OCH(CH₃)₂, -O-CH₂-CH₂-O-CH₃, -N(CH₂-CH₂-OH)₂, -N(CH₂-CHOH-CH₃)₂, Morpholino, -N(CH₂-CH₂-OH)CH₂-CH₂-CONH₂, sowie die Reste der Formel 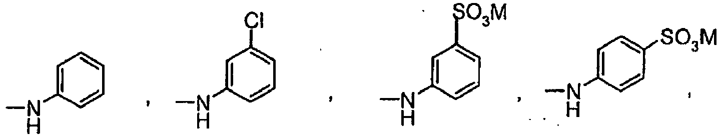 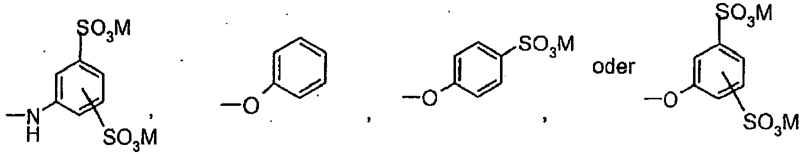 wobei
- M: die oben angegebene Bedeutung besitzt.

Ganz besonders bevorzugt stehen die Reste R¹ bis R⁴ unabhängig voneinander für 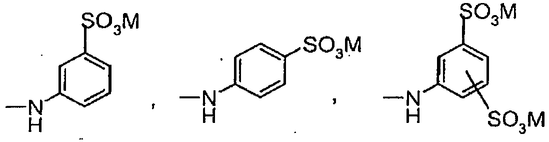 -NH-CH₂CH₂OH, -N(CH₂-CH₂-OH)₂, -N(CH₂-CHOH- CH₃)₂, Anilino oder Morpholino.

Bevorzugt beträgt die Summe von Aufheller der Formel Ia und VII in der Zubereitung mehr als 70 Gew.-%, insbesondere mehr als 80 Gew.-%, bezogen auf den Gesamtaufhellergehalt.

Besonders bevorzugt enthält die Zubereitung eine Mischung von Aufhellern, enthaltend wenigstens eine Verbindung der Formel Ia und wenigstens eine Verbindung der Formel VII. Ganz besonders bevorzugt haben die Reste R¹ bis R⁴ und M jeweils in Formel Ia und VII die gleiche Bedeutung.

Hinsichtlich der Anteile der Formel Ia an dieser Mischung gelten die obigen Angaben.

Als Carriersubstanzen kommen generell hydrophile Polymere mit der Fähigkeit zur Ausbildung von Wasserstoffbrückenbindungen in Frage. Bevorzugte Carriersubstanzen sind Polyvinylalkohole, Carboxymethylcellulosen sowie Polyethylenglykole mit einem zahlenmittleren Molgewicht von 200 bis 8000 g/mol, wie auch beliebige Mischungen dieser Substanzen, wobei diese Polymere gegebenenfalls modifiziert sein können. Bevorzugte Polyvinylalkohole sind solche mit einem Hydrolysegrad > 85%, bevorzugte Carboxymethylcellulosen solche mit einem Substitutionsgrad DS von > 0,5. Besonders bevorzugt sind Polyethylenglykole mit einem zahlenmittleren Molgewicht Mₙ von 200 bis 8000 g/mol.

Weiterhin kommen z.B. native, derivatisierte oder abgebaute Stärken, Alginate, Casein, Proteine, Polyacrylamide, Hydroxyalkylcellulose sowie Polyvinylpyrrolidon in Frage.

Zubereitungen, die zusätzlich zu den erfindungsgemäß verwendeten Triazinylflavonataufhellern bereits bekannte Aufheller aus der Gruppe der Triazinylflavonataufheller enthalten, können, bezogen auf ihre Aufheller-wirksamen Komponenten, beispielsweise dadurch hergestellt werden, dass die erfindungsgemäß verwendeten Triazinylflavonataufheller als Reinsubstanzen oder in Form von Lösungen ihrer Reinsubstanzen geeigneter Konzentration zu den bekannten Triazinylflavonataufhellern hinzugemischt werden.

Als Vorteile der Zubereitung zeichnen sich die Zubereitungen beispielsweise bei Anwendung im pigmentierten Strich auf Papier bei extinktionsgleichem Einsatz - verglichen mit Triazinylflavonataufhellerzubereitungen, welche die erfindungsgemäß verwendeten Triazinylflavonataufheller nicht enthalten, ansonsten aber gleiche Zusammensetzung aufweisen - durch ein verbessertes Weißeaufbauverhalten und einen höheren maximal erreichbaren Weißgrad aus, so dass z.B. auf diese Weise bei gleichem Einsatz ein größerer Aufhelleffekt erreicht werden kann. Alternativ dazu kann gewünschtenfalls mit einem geringeren Einsatz ein gleich hoher Aufhellef fekt erreicht werden.

Die Erfindung betrifft weiterhin die mit den erfindungsgemäß verwendeten Aufhellern bzw. ihren Zubereitungen aufgehellten Streichmassen enthaltend
- Wasser,
- wenigstens ein Weißpigment,
- wenigstens einen Binder, insbesondere Latex-Binder, und
- wenigstens einen Aufheller der Formel Ia oder eine einen Aufheller der Formel Ia enthaltende Zubereitung.

Bevorzugt beträgt die Menge an Binder, insbesondere Latex-Binder (gerechnet als Trockensubstanz) 3 bis 20 Gew.-%, insbesondere 5 bis 15 Gew.-%, unabhängig davon die Menge an gegebenenfalls eingesetztem davon verschiedenem synthetischen Cobinder 0,1 bis 3 Gew.-%, insbesondere 0,5 bis 1,5 Gew.-% und ebenfalls unabhängig davon die Menge an Aufheller der Formel Ia oder der diesen enthaltenden Zubereitung, bezogen auf aufhellerwirksame Komponenten, 0,025 bis 1 Gew.-%, jeweils bezogen auf die Menge an Weißpigment.

Bevorzugt enthält die Streichmasse zusätzlich wenigstens ein Dispergiermittel, insbesondere in einer Menge von 0,05 bis 1 Gew.-%, bezogen auf das Weißpigment in der Streichmasse. Als Dispergiermittel kommt vorzugsweise Polyacrylsäure bzw. entsprechende Salze in Frage. Der Wassergehalt der Streichmasse beträgt bevorzugt 30 bis 50 Gew.-%, bezogen auf die Gesamtmenge an Streichmasse.

Als Weißpigmente werden üblicherweise Calciumcarbonat in natürlicher oder gefällter Form, Kaolin, Talkum, Titandioxid, Satinweiss, Aluminiumhydroxid und Bariumsulfat eingesetzt, auch in Form von Mischungen.

Als Latexbinder kommen alle gebräuchlichen Latexbinder, die zur Herstellung von Papierstreichmassen verwendet werden, in Frage. Als davon verschiedene synthetische Cobinder enthalten die Streichmassen beispielsweise Carboxymethylcellulose, Hydroxyalkylcellulose und/oder Polyvinylalkohol sowie synthetische Verdicker auf Acrylatbasis.

### Herstellungsbeispiele

### Beispiel 1 Herstellung einer Verbindung der Formel VIII

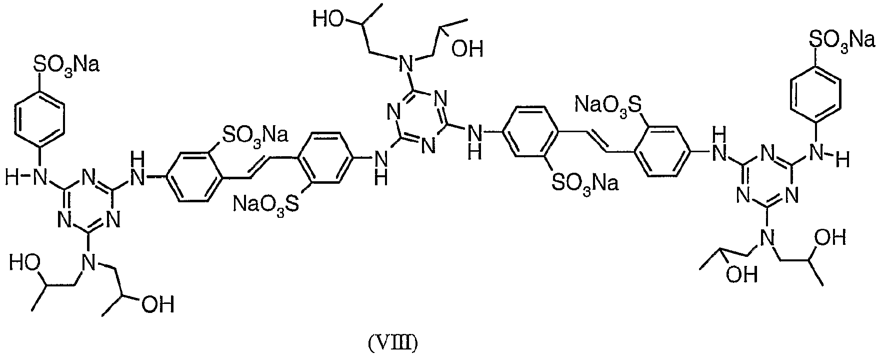

### Stufe 1:

Zu einer Emulgator-haltigen, gerührten Suspension von 0,54 mol Cyanurchlorid in 600 g Wasser von 8°C dosiert man innerhalb ca. 1 h eine Lösung von 0,53 mol Natriumsulfanilat in 460 g Wasser, während man den pH-Wert durch gleichzeitiges Zudosieren von 15 %iger Sodalösung zwischen 2,1 und 2,3 und die Temperatur unterhalb 25°C hält. Nach beendeter Zudosierung rührt man ca. 40 min unterhalb 25°C im angegebenen pH-Bereich nach. Die Gesamtmenge an benötigter Sodalösung beträgt ca. 187 g.

Anschließend stellt man den pH-Wert mit 15 %iger Sodalösung auf ca. 6,8 und dosiert innerhalb ca. 1 h eine wässrige Lösung von 0,52 mol des Natriumsalzes der 4-Amino-4'-nitrostilben-2,2'-disulfonsäure ein, während man die Reaktionsmischung gleichzeitig auf ca. 35°C erwärmt und den pH-Wert durch Zudosieren von 15 %iger Sodalösung bei 6,8 hält. Nach beendeter Dosierung heizt man unter weiterem Halten des pH-Werts bis auf 50°C und rührt unter diesen Bedingungen 20 min nach. Es werden ca. 184 g 15 %ige Sodalösung verbraucht.

Zur Reaktionsmischung lässt man bei ca. 50°C beginnend innerhalb 15 min 0,68 mol wässrige, 84 %ige Diisopropanolaminlösung laufen, während man gleichzeitig auf 100°C aufheizt. Der pH-Wert fällt ab und wird durch Eindosieren von 15 %iger Sodalösung bei 7,4 gehalten. Es wird bei pH 7,4 und 100°C 3 h nachgerührt.

Man erhält eine wässrige, salzhaltige Lösung, welche etwa 0,5 mol der Verbindung der Formel VI mit 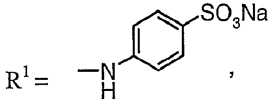 und R²= -N(CH₂-CHOH-CH₃)₂ enthält

### Stufe 2:

Zu einer gerührten Suspension von ca. 7 mol Eisenpulver (Qualität "Zur Reduktion") in 800 g Wasser von 90-100°C tropft man zügig ca. 380 g 37 %ige Salzsäure, wobei sich kurzzeitig ein pH-Wert von unter 1 einstellt. Anschließend lässt man innerhalb ca. 1 h 0,5 mol der Verbindung der Formel VI mit 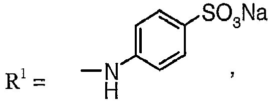 und R² = -N(CH₂-CHOH-CH₃)₂ in Form der oben beschriebenen wässrigen Lösung der Stufe 1 zulaufen. Nach beendeter Dosierung der Stufe 1 rührt man noch 1,5 h bei 98-100°C nach, lässt auf 80°C abkühlen und filtriert vom Eisenschlamm ab.

Das Filtrat wird auf 30°C abgekühlt. Unter Rühren tropft man bei dieser Temperatur 1800 g 15 %ige Sodalösung zu, wodurch der pH-Wert bis auf ca. 9 steigt. Das ausgefallene basische Eisencarbonat wird abfiltriert. Man stellt den pH-Wert des Filtrats durch Eindosieren von 900 g 37 %iger Salzsäure auf 2,0-2,2 ein, gibt 500g Natriumchlorid hinzu und verrührt über Nacht.

Das auskristallisierte Produkt wird über eine Nutsche isoliert und 20 h trocken gesaugt. Anschließend wird es in 1400 g Wasser eingetragen und aufgerührt, wodurch sich eine dicke Suspension bildet. Man stellt den pH-Wert mit 292 g 15 %iger Sodalösung auf 7-8, verrührt bis zur fast klaren Lösung und filtriert. Man erhält eine Lösung, die 0,34 mol der Verbindung der Formel II mit 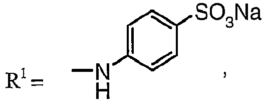 und R²= -N(CH₂-CHOH-CH₃)₂ enthält.

### Stufe 3:

Zu einer gerührten Suspension von 0,17 mol Cyanurchlorid in 500 g emulgatorhaltigem Wasser von 8°C dosiert man innerhalb ca. 30 min eine Lösung von 0,17 mol der oben beschriebenen Lösung der Stufe 2, während man den pH-Wert durch gleichzeitiges Zudosieren von 15 %iger Sodalösung zwischen 4 und 4,5 und die Temperatur unterhalb 25°C hält. Die Gesamtmenge an benötigter Sodalösung beträgt ca. 60 g.

Anschließend stellt man den pH-Wert mit 15 %iger Sodalösung auf ca. 6,8 und dosiert innerhalb ca. 1 h weitere 0,17 mol der oben beschriebenen Lösung der Stufe 2 zu, während man die Reaktionsmischung gleichzeitig auf ca. 35°C erwärmt und den pH-Wert durch Zudosieren von 15 %iger Sodalösung bei 6,8 hält. Nach beendeter Dosierung heizt man unter weiterem Halten des pH-Werts bis auf 50°C und rührt unter diesen Bedingungen 20 min nach. Es werden ca. 60 g 15 %ige Sodalösung verbraucht.

### Stufe 4:

Zur Reaktionsmischung lässt man bei ca. 50°C beginnend innerhalb 15 min 0,23 mol wässrige, 84 %ige Diisopropanolaminlösung laufen, während man gleichzeitig auf 100°C aufheizt. Der pH-Wert fällt ab und wird durch Eindosieren von 15 %iger Sodalösung bei 7,4 abgefangen. Nach Erreichen von pH 7,4 wird 3 h bei pH 7,4 und 100°C nachgerührt.

Man erhält eine wässrige, salzhaltige Lösung, welche etwa 0,17 mol der Verbindung der Formel VIII enthält.

### Beispiel 2

Setzt man in Stufe 4 des Beispiels 1 anstatt 0,23 mol 84 %iger Diisopropanolaminlösung 0,23 mol 90 %ige Diethanolaminlösung ein und verfährt ansonsten weiter wie in Beispiel 1, Stufe 4 beschrieben, erhält man eine wässrige, salzhaltigen Lösung, welche etwa 0,17 mol der Formel IX enthält.

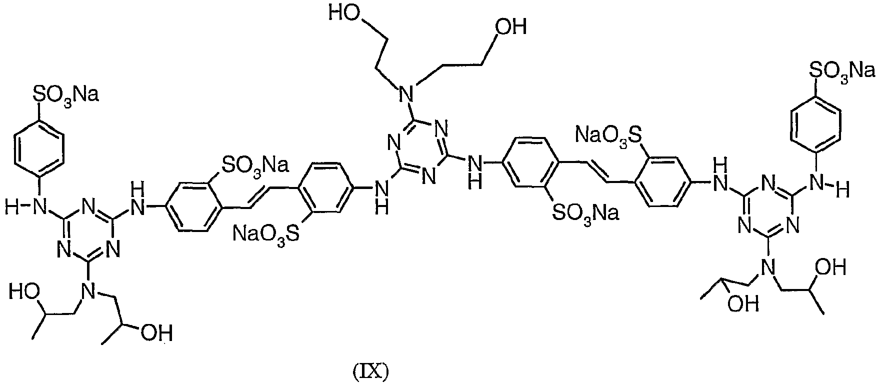

### Beispiel 3

Setzt man in Stufe 1 des Beispiels 1 statt 0,68 mol 84 %iger Diisopropanolaminlösung 0,68 mol Diethanolaminlösung 90 %ig ein und verfährt ansonsten weiter wie in Beispiel 1, Stufe 2 bis 4 beschrieben, erhält man eine wässrige, salzhaltige Lösung, welche etwa 0,15 mol der Verbindung der Formel X enthält.

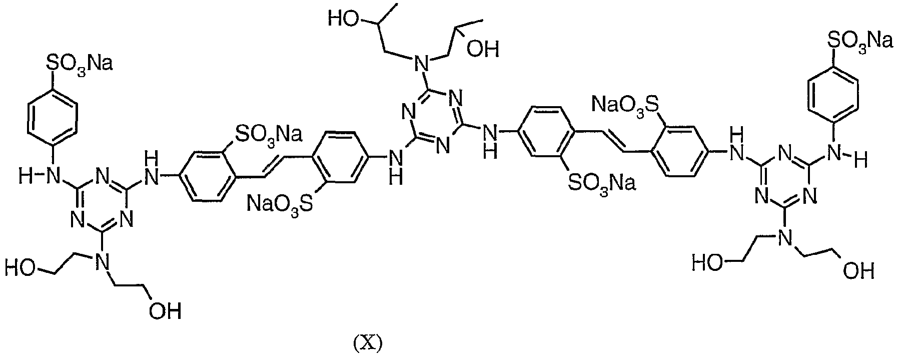

### Beispiel 4

Setzt man in Stufe 4 des Beispiels 3 anstatt 0,23 mol 84 %ige Diisopropanolaminlösung 0,23 mol 90 %ige Diethanolaminlösung ein und verfährt ansonsten weiter wie in Beispiel 1 beschrieben, erhält man eine wässrige, salzhaltige Lösung, welche etwa 0,16 mol der Verbindung der Formel XI enthält

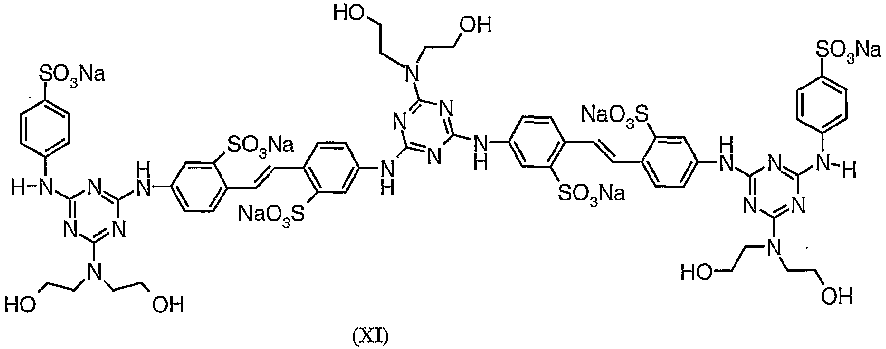

### Beispiel 5

Dosiert man in Beispiel 1, Stufe 3, bei pH 6,8 nicht weitere 0,17 mol Lösung der Stufe 2 zu, sondern stattdessen eine äquimolare Lösung, die gemäß Beispiel 3, Stufe 2 hergestellt wurde, und verfährt weiter wie in Beispiel 1, Stufe 4 beschrieben, erhält man eine wässrige, salzhaltige Lösung, welche etwa 0,15 mol der Verbindung der Formel XII enthält.

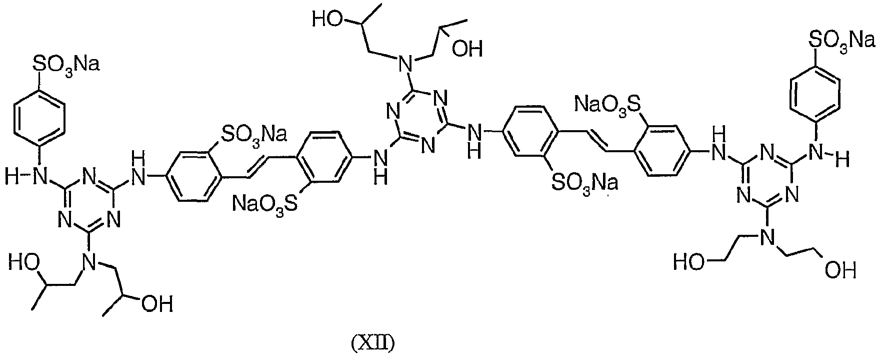

### Vergleichsbeispiel 1 (entspricht Bsp. 4. aus EP-A-1 355 004)

77,6 g eines membranfiltrierten wässrigen Konzentrats mit einem E1/1-Wert von 161 und einem pH-wert von 8,5, das den Aufheller der Formel VII mit R₁ = R₃ = über das Stickstoffatom gebundener Rest des Natriumsalzes der p-Sulfanilsäure und R₂ = R₄ = über das Stickstoffatom gebundener Rest des Diethanolamins enthält, werden unter Rühren bei Raumtemperatur mit 22g demineralisiertem Wasser versetzt und mit ca. 10%iger Natronlauge auf pH 9,0 gestellt. Man erhält eine carrierfreie Aufhellerpräparation mit einem E1/1-Wert von 125 in Form einer gelbbräunlichen homogenen Flüssigkeit. Dies entspricht einem Gehalt an Aufheller von ca. 21%

### Verzleichsbeispiel 2 (entspricht Beispiel 1 aus EP-A-1 355 004)

Man verfährt wie in Vergleichsbeispiel 1, setzt aber einen Aufheller der Formel VII mit R₁ = R₃ = über das Stickstoffatom gebundener Rest des Natriumsalzes der p-Sulfanilsäure und R₂ = R₄ = über das Stickstoffatom gebundener Rest des Diisopropanolamins ein. Man erhält eine carrierfreie Aufhellerpäparation mit einem E1/1-Wert von 125. Dies entspricht einem Gehalt des Aufhellers von ca. 23 Gew. %

### Anwendungsbeispiele

### Anwendungsbeispiel 1:

Eine Papierstreichmasse wird aus folgenden Komponenten hergestellt:
379 Teile Kreide Hydrocarb 90
162 Teile Clay SPS
108,0 Teile eines Styrol-Butadien-Latex 50 %ig
27 Teile Polyvinylalkohol als Cobinder (20 %ig)
3,6 Teile Teile Polysalz S als Dispergiermittel (50 %ig) (Basis Polyacrylsäure, BASF AG)
320,7 Teile Wasser
5 %ige Natronlauge.

Man wählt die Natronlaugemenge so, dass ein pH-Wert von 8,8 resultiert.

Die Streichmasse wird in 10 Teile geteilt und je ein Teil mit 0,2 %, 0,4 %, 0,8 %, 1,2 % und 1,6 Gew.-% einer mittels Membranfiltration entsalzten und auf einen E1/1-Wert von 125 (≙ 21 Gew.-% Aufhellergehalt, bezogen auf Präparation) aufkonzentrierten Aufhellerpräparation aus Herstellungsbeispiel 4 versetzt und dann 10 min verrührt. Die Zugabemengen beziehen sich dabei auf den Weißpgimentgehalt der Streichmasse. Zum Vergleich wird je ein Teil der Streichmasse auf dieselbe Weise mit gleichen Mengen der Aufhellerpräparation aus Vergleichsbeispiel 1 versetzt. Die erhaltenen, aufgehellten Streichmassen werden mit einem Laborrakelgerät (Firma Erichsen, K-Control-Coater, Modell K202) auf holzfreie Papiere mit einem Flächengewicht von ca. 80g/m² aufgetragen. Die gestrichenen Papiere werden 1 min bei 95°C auf einem Trockenzylinder getrocknet und danach für 3h bei 23°C und 50 % relativer Feuchte gelagert. Das Strichauftragsgewicht wurde mit 15 g/m²ermittelt. Die Messung der Parameter L*, a*, b* und die Ermittlung des CIE-Weißgrades wird sodann mit einem Weißgradmessgerät (Datacolor Elrepho 2000) aufgenommen.

Die erhaltenen Werte sind in den Tabellen 1 und 2 aufgelistet

**Tabelle 1: Aufhellerpräparation aus Bsp. 4 (E1/1=125)**

| **Menge (%)** | **CIE-Weißgrad** | **L*** | **a*** | **b*** |
|---|---|---|---|---|
| 0,2 | 99,37 | 94,60 | 0,86 | -2,79 |
| 0,4 | 104,12 | 94,67 | 1,11 | -3,81 |
| 0,8 | 107,5 | 94,85 | 1,01 | -4,37 |
| 1,2 | 107,09 | 94,96 | 0,63 | -4,33 |
| 1,6 | 103,41 | 95,05 | -0,15 | -3,47 |

**Tabelle 2: Aufhelleipräparation aus Vergleichsbeispiel 1 (E1/1=125)**

| **Menge (%)** | **CIE-Weißgrad** | **L*** | **a*** | **b*** |
|---|---|---|---|---|
| 0,2 | 97,49 | 94,55 | 0,74 | -2,41 |
| 0,4 | 101,80 | 94,57 | 0,95 | -3,35 |
| 0,8 | 104,97 | 94,78 | 0,93 | -3,94 |
| 1,2 | 106,37 | 94,94 | 0,74 | -4,17 |
| 1,6 | 105,73 | 95,03 | 0,46 | -3,99 |

Man sieht, dass bei jeweils E1/1-gleichem Einsatz der erfindungsgemäße Aufheller in der PVA-haltigen Streichfarbe im Bereich kleiner Einsatzkonzentrationen zu besseren CIE-Weißgradwerten führt als der aus dem nicht erfindungsgemäßen Vergleichsbeispiel 1.

### Anwendungsbeispiel 2:

Man verfährt wie in Anwendungsbeispiel 1, setzt jedoch einerseits eine mittels Membranfiltration entsalzte und auf einen E1/1-Wert von 125 (≙ ca. 23 Gew.-% Aufheller, bezogen auf Aufhellerpräparation) aufkonzentrierte Aufhellerpräparation aus Herstellungsbeispiel 1 andererseits eine gleiche Menge Aufhellerpräparation aus Vergleichsbeispiel 2 ein.

Die erhaltenen Werte sind in den Tabellen 3 und 4 aufgelistet.

**Tabelle 3: Aufhellerpräparation aus Bsp. 1 (E1/1=125)**

| **Menge (%)** | **CIE-Weißgrad** | **L*** | **a*** | **b*** |
|---|---|---|---|---|
| 0,2 | 99,10 | 94,58 | 0,83 | -2,75 |
| 0,4 | 103,89 | 94,63 | 1,1 | -3,78 |
| 0,8 | 107,68 | 94,81 | 1,17 | -4,53 |
| 1,2 | 108,07 | 94,85 | 0,97 | -4,60 |
| 1,6 | 107,59 | 94,84 | 0,67 | -4.,49 |

**Tabelle 4: Aufhellerpräparation aus Vergleichsbeispiel 2 (E1/1=125)**

| **Menge (%)** | **CIE-Weißgrad** | **L*** | **a*** | **b*** |
|---|---|---|---|---|
| 0,2 | 97,66 | 94,58 | 0,77 | -2,43 |
| 0,4 | 101,92 | 94,63 | 0,98 | -3,34 |
| 0,8 | 106,47 | 94,74 | 1,12 | -4,30 |
| 1,2 | 108,72 | 94,75 | 1,09 | -4,79 |
| 1,6 | 109,65 | 94,99 | 0,90 | -4,88 |

Man sieht, dass bei jeweils E1/1-gleichem Einsatz der erfindungsgemäße Aufheller in der PVAhaltigen Streichfarbe im Bereich kleiner Einsatzkonzentrationen zu besseren CIE-Weißgradwerten führt als der aus dem Vergleichsbeispiel 2.

### Anwendungsbeispiel 3:

Man verfährt wie in Anwendungsbeispiel 1, setzt jedoch einerseits eine durch Vermischung hergestellte Zubereitung aus 5 Gewichtsteilen einer mittels Membranfiltration entsalzten und auf einen E1/1-Wert von 125 aufkonzentrierte Aufhellerpräparation aus Herstellungsbeispiel 4 und 95 Gewichtsteilen der Aufhellerpräparation des Vergleichsbeispiels 1, andererseits eine gleiche Menge Aufhellerpräparation aus Vergleichsbeispiel lein.

Die erhaltenen Werte sind in den Tabellen 5 und 6 aufgelistet.

**Tabelle 5: Zubereitung aus 5 Gew.-% entsalzter und membranfiltrierter Aufhellerpräparation aus Herstellungsbeispiel 4 (E1/1=125) und 95 Gew. % Aufhellerpräparation aus Vergleichsbeispiel 1 (E1/1=125)**

| **Menge (%)** | **CIE-Weißgrad** | **L*** | **a*** | **b*** |
|---|---|---|---|---|
| 0,2 | 97,30 | 94,49 | 0,72 | -2,39 |
| 0,4 | 101,98 | 94,65 | 0,94 | -3,35 |
| 0,8 | 106,61 | 94,81 | 1,04 | -4,29 |
| 1,2 | 107,28 | 94,85 | 0,83 | -4,47 |
| 1,6 | 107,83 | 94,88 | 0,63 | -4,53 |

**Tabelle 6: Aufhellerpräparation aus Vergleichsbeispiel 1 (E1/1=125)**

| **Menge (%)** | **CIE-Weißgrad** | **L*** | **a*** | **b*** |
|---|---|---|---|---|
| 0,2 | 97,49 | 94,55 | 0,74 | -2,41 |
| 0,4 | 101,80 | 94,57 | 0,95 | -3,35 |
| 0,8 | 104,97 | 94,78 | 0,93 | -3,94 |
| 1,2 | 106,37 | 94,94 | 0,74 | -4,17 |
| 1,6 | 105,73 | 95,03 | 0,46 | -3,99 |

Man sieht, dass die erfindungsgemäße Zubereitung gegenüber der nicht erfindungsgemäßen Aufhellerpräparation insgesamt eine bessere Aufhellwirkung zeigt.

### Anwendungsbeispiel 4:

Man verfährt wie in Anwendungsbeispiel 1, setzt jedoch einerseits eine durch Vermischung hergestellte Zubereitung aus 5 Gewichtsteilen einer mittels Membranfiltration entsalzten und auf einen E1/1-Wert von 125 aufkonzentrierte Aufhellerpräparation aus Herstellungsbeispiel 1 und 95 Gewichtsteilen der Aufhellerpräparation des Vergleichsbeispiel 2, andererseits eine gleiche Menge Aufhellerpräparation aus Vergleichsbeispiel 2 ein.

Die erhaltenen Werte sind in den Tabellen 7 und 8 aufgelistet.

**Tabelle 7: Zubereitung aus 5 Gew.-% entsalzter und membranfiltrierter Aufhellerpräparation aus Herstellungsbeispiel 1 (E1/1=125) und 95 Gew.-% Aufhellerpräparation aus Vergleichsbeispiel 2 (E1/1=125)**

| **Menge (%)** | **CIE-Weißgrad** | **L*** | **a*** | **b*** |
|---|---|---|---|---|
| 0,2 | 98,13 | 94,49 | 0,80 | -2,58 |
| 0,4 | 102,97 | 94,62 | 1,06 | -3,58 |
| 0,8 | 107,60 | 94,78 | 1,15 | -4,42 |
| 1,2 | 109,36 | 94,80 | 1,12 | -4,91 |
| 1,6 | 111,04 | 94,89 | 1,06 | -5,24 |

**Tabelle 8: Aufhellerpräparation aus Vergleichsbeispiel 2 (E1/1=125)**

| **Menge (%)** | **CIE-Weißgrad** | **L*** | **a*** | **b*** |
|---|---|---|---|---|
| 0,2 | 97,66 | 94,58 | 0,77 | -2,43 |
| 0,4 | 101,92 | 94,63 | 0,98 | -3,34 |
| 0,8 | 106,47 | 94,74 | 1,12 | -4,30 |
| 1,2 | 108,72 | 94,75 | 1,09 | -4,79 |
| 1,6 | 109,65 | 94,99 | 0,90 | -4,88 |

Man sieht, dass die erfindungsgemäße Zubereitung gegenüber der nicht erfindungsgemäßen Aufhellerpräparation insgesamt eine bessere Aufhellwirkung zeigt.

Die Zubereitungen der Anwendungsbeispiele besitzen auch nach Bestimmung der relevanten Flächenanteile der Aufheller durch die in der Beschreibung beschriebenen HPLC-Methoden die gleichen Verhältnisse wie sie der Einwaage entsprechen (95:5).

## Patentansprüche

1. Verwendung einer Verbindung der Formel Ia 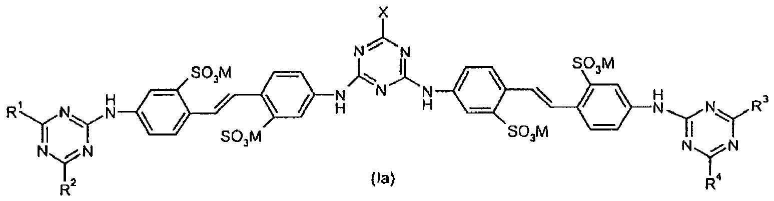 worin
R¹ und R³ unabhängig voneinander für einen Rest der Formel 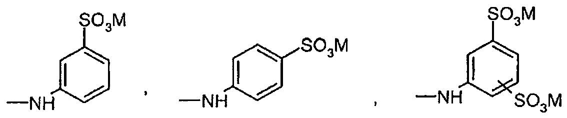 steht, worin
M für Wasserstoff, ein Äquivalent eines ein- oder zweiwertigen Metallions oder eines gegebenenfalls organisch substituierten Ammoniumions steht,
R² und R⁴ unabhängig voneinander für -NHCH₂-CH₂OH, -N(CH₂CH₂OH)₂, 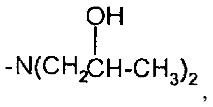 Anilino oder Morpholino steht und
X für -OH oder für -NHCH₂CH₂OH, -N(CH₂CH₂OH)₂, 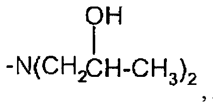 Anilino oder Morpholino steht,
als Aufheller in Papierstreichmassen.

2. Verwendung gemäß Anspruch 1, **dadurch gekennzeichnet, dass** M für ein Äquivalent eines ein- oder zweiwertigen Metallions aus der Gruppe der Alkali- oder Erdalkalimetalle steht.

3. Verwendung gemäß Anspruch 1 oder 2, **dadurch gekennzeichnet, dass** R¹ = R³ und R² = R⁴ ist.

4. Verwendung nach wenigstens einem der Ansprüche 1 bis 3, **dadurch gekennzeichnet, dass** man Verbindungen der Formel Ia herstellt, indem man eine Verbindung der Formel II 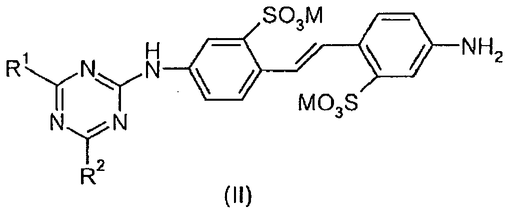 worin
R¹, R² und M die in wenigstens einem der Ansprüche 1 bis 3 angegebene Bedeutung haben, mit einer Verbindung der Formel III
Y-B¹-Z (III)
worin
Y und Z unabhängig voneinander für Abgangsgruppen stehen, die durch die freie Aminogruppe der Verbindungen der Formel II ersetzt werden können und B¹ für einen Rest der Formel 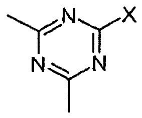 steht und X die obige Bedeutung hat, zu einer Verbindung der Formel IIIa umsetzt 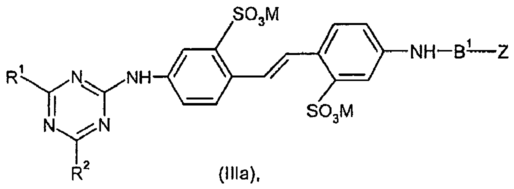 worin
R¹, R², B¹ und Z die oben genannte Bedeutung haben,
und die Verbindung IIIa weiter mit einer Verbindung der Formel IV 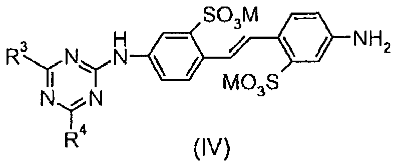 worin R³, R⁴ und M die oben beschriebene Bedeutung haben, zur Verbindung der Formel Ia umsetzt.

5. Verwendung nach wenigstens einem der Ansprüche 1 bis 3, **dadurch gekennzeichnet, dass** die Verbindung der Formel Ia in einer Zubereitung enthalten ist, enthaltend, bezogen auf den Gesamtaufhellergehalt, 1 bis 15 Flächen-% wenigstens einer Verbindung nach wenigstens einem der Ansprüche 1 bis 3, wobei der prozentuale Flächenanteil in einem Hochdruckflüssigkeitschromatogramm bei einer Wellenlänge von 350 nm an einer wässrigen Messlösung der Zubereitung gemessen wird, wobei die für 100 ml Messlösung einzuwiegende, in Gramm ausgedrückte Menge der Zubereitung, multipliziert mit ihrem E1/1-Wert, 50 betragen soll, wobei der E1/1-Wert dem fiktiven, auf eine 1%ige Lösung der zu bestimmenden Probe hochgerechneten Extinktionswert entspricht.

6. Verwendung nach Anspruch 5, **dadurch gekennzeichnet, dass** die Zubereitung Wasser und 5 bis 50 Gew.-% Gesamtaufheller enthält.

7. Verwendung nach wenigstens einem der Ansprüche 5 oder 6, **dadurch gekennzeichnet, dass** in der Zubereitung als Aufheller eine Mischung aus der Verbindung der Formel Ia nach wenigstens einem der Ansprüche 1 bis 3 und der Verbindung VII eingesetzt wird 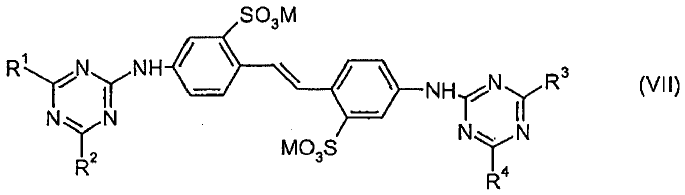 worin
R¹ bis R⁴ und M unabhängig von der Bedeutung in Formel Ia die Bedeutung besitzen, dass
R¹, R², R³ und R⁴ jeweils unabhängig voneinander für OR⁵ oder NR⁶R⁷ stehen, wobei
R⁵, R⁶ und R⁷ jeweils unabhängig voneinander für Wasserstoff, substituiertes oder unsubstituiertes Alkyl, oder substituiertes oder unsubstituiertes Aryl stehen, wobei
R⁶, R⁷ zusammen mit dem N-Atom, an das sie gebunden sind und gegebenenfalls weiteren Heteroatomen auch einen aliphatischen oder aromatischen Ring bilden können, und
M die Bedeutung gemäß Anspruch 1 oder 2 besitzt.

8. Verwendung nach Anspruch 7, **dadurch gekennzeichnet, dass** in der Verbindung VII wenigstens einer der Reste R¹ oder R² die gleiche Bedeutung hat wie wenigstens einer der Reste R³ oder R⁴.

9. Verwendung nach Anspruch 7 oder 8, **dadurch gekennzeichnet, dass** in der Verbindung VII die Reste R¹, R², R³ und R⁴ unabhängig voneinander für Phenoxy, mono- oder disulfoniertes Phenoxy, Phenylamino, mono- oder disulfoniertes Phenylamino, Phenylamino substituiert durch C₁-C₃-Alkyl, Cyano, Halogen, -COOR, -CONH-R, -NH-COR, -SO₂NH-R oder -OR; weiterhin für die Reste Morpholino, Piperidino, Pyrrolidino, -OC₁-C₄-Alkyl, -NH-(C₁-C₄-Alkyl), -N(C₁-C₄-Alkyl)₂, -NH(C₂-C₄-Alkylen)-OR, -N[(C₂-C₄-Alkylen)-OR]₂, -NH(C₂-C₄-Hydroxyalkyl), -N(C₂-C₄-Hydroxyalkyl)₂, -NH(C₂-C₄-Alkylen-O-C₂-C₄-Alkylen-OR), eine Aminosäure bzw. ein Aminosäuresalz oder ein Aminosäureamid, von deren Aminogruppe ein Wasserstoffatom entfernt wurde, -N(CH₃)(CH₂CH₂OH), -NH₂, -OCH₂CH₂SO₃M, -NH-CH₂CH₂SO₃M, -N(CH₂CH₂SO₃M)₂ oder -N(CH₂CH₂OH)CH₂CH₂CONH₂ stehen, wobei R=H oder C₁-C₃-Alkyl bedeutet und M die oben angegebene Bedeutung hat.

10. Verwendung nach wenigstens einem der Ansprüche 7 bis 9, **dadurch gekennzeichnet, dass** in der Verbindung VII die Reste R¹ bis R⁴ unabhängig voneinander für -NH₂, -NH-CH₃, -NH-C₂H₅, -N(CH₃)₂, -N(C₂H₅)₂, -NH-C₂-C₄-Hydroxyalkyl, -NH-CH₂CH₂SO₃M, -NH-CH₂-CH₂-O-CH₂-CH₂-OH, -OCH₃, -OCH(CH₃)₂, -O-CH₂-CH₂-O-CH₃, -N(CH₂-CH₂-OH)₂, -N(CH₂-CHOH-CH₃)₂, Morpholino, -N(CH₂-CH₂-OH)CH₂-CH₂-CONH₂ sowie die Reste der Formel 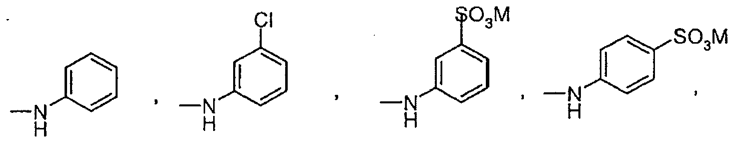 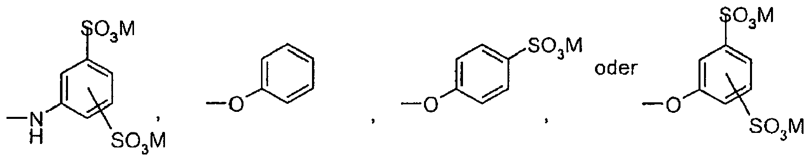 stehen, wobei M die oben angegebene Bedeutung besitzt.

11. Verwendung nach wenigstens einem der Ansprüche 7 bis 10, **dadurch gekennzeichnet, dass** in der Verbindung VII die Reste R¹ bis R⁴ unabhängig voneinander für 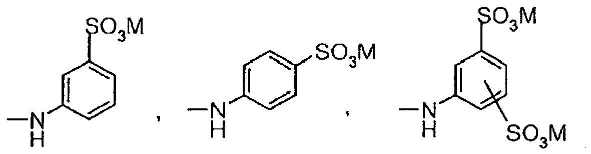 -NH-CH₂CH₂OH, -N(CH₂-CH₂-OH)₂, -N(CH₂-CHOH-CH₃)₂, Anilino oder Morpholino stehen.

12. Verwendung nach wenigstens einem der Ansprüche 1 bis 3, **dadurch gekennzeichnet, dass** die Verbindung der Formel Ia in einer Streichmasse enthalten ist, enthaltend
- Wasser,
- wenigstens ein Weißpigment,
- wenigstens einen Binder und
- wenigstens einen Aufheller wie in Anspruch 1 definiert oder eine Zubereitung wie in wenigstens einem der Ansprüche 5 bis 11 definiert.

13. Verwendung nach Anspruch 12, **dadurch gekennzeichnet, dass** der Binder ein Latex-Binder ist.

14. Streichmasse, enthaltend
- Wasser,
- wenigstens ein Weißpigment,
- wenigstens einen Binder und
- wenigstens einen Aufheller wie in Anspruch 1 definiert oder eine Zubereitung wie in wenigstens einem der Ansprüche 5 bis 11 definiert.

15. Streichmasse nach Anspruch 14, **dadurch gekennzeichnet, dass** der Binder ein Latex-Binder ist.

## Claims

1. Use of a compound of formula Ia 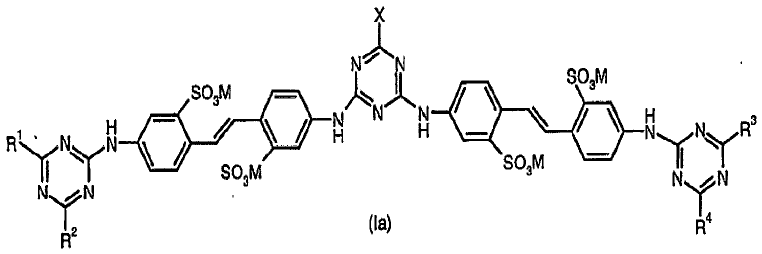 wherein
R¹ and R³, independently of each other, stand for a group of the formula 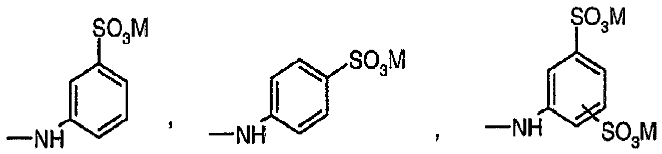 wherein
M stands for hydrogen, one equivalent of a mono- or divalent metal ion, or an optionally organic substituted ammonium ion,
R² and R⁴ independently of each other, stand for -NHCH₂-CH₂OH, -N(CH₂CH₂OH)₂, 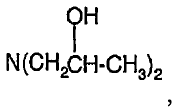 anilino, or morpholino, and X stands for OH or for -NHCH₂CH₂OH, -N(CH₂CH₂OH)₂, 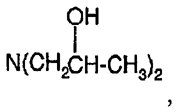 anilino, or morpholino,
as brightener in paper coating compositions.

2. The use according to claim 1, **characterized in that** M stands for one equivalent of a mono- or divalent metal ion from the group of alkali or alkaline-earth metals.

3. The use according to claim 1 or 2, **characterized in that** R¹ = R³ and R² = R⁴.

4. The use according to at least one of claims 1 to 3, **characterized in that** compounds of the formula Ia are prepared **in that** a compound of formula II 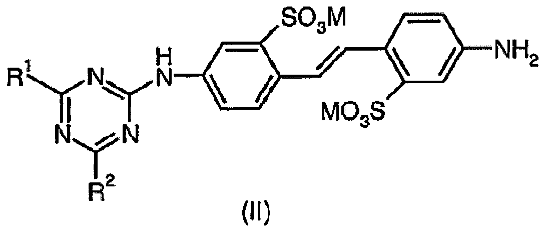 wherein
R¹, R² and M have the meaning as defined in at least one of claims 1 to 3, is reacted with a compound of formula (III)
Y-B¹-Z (III)
wherein
Y and Z independently of each other, stand for leaving groups that can be replaced by the free amino group of the compounds of formula II, and B¹ stands for a group of formula 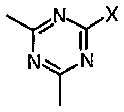 and X has the meaning as defined above, to a compound of formula IIIa 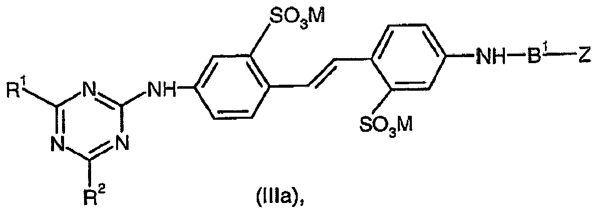
wherein
R¹, R², B¹ and Z have the meaning as defined above,
and the compound IIIa is further reacted with a compound of formula IV 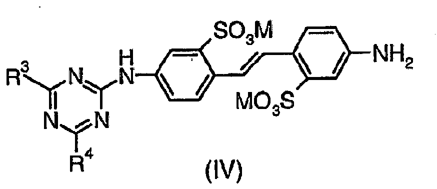 wherein R3, R⁴ and M have the meaning as defined above, into the compound of the formula Ia.

5. The use according to at least one of claims 1 to 3, **characterized in that** the compound of formula Ia is contained in a preparation containing, based on the total brightener content, 1 to 15 area% of at least one compound according to at least one of claims 1 to 3, wherein the area% is measured in a high-pressure liquid chromatogram at a wavelength of 350 nm on an aqueous measurement solution of the preparation, wherein the amount of preparation expressed in grams weighed in per for 100 ml of measurement solution multiplied by its E1/1 value should be 50, wherein the E1/1 value corresponds to the fictitious extinction value extrapolated to a 1% solution of the sample being determined.

6. The use according to claim 5, **characterized in that** the preparation contains water and 5 to 50 wt% total brightener.

7. The use according to at least one of claims 5 or 6, **characterized in that** in the preparation a mixture of the compound of formula Ia according to at least one of claims 1 to 3 and the compound VII 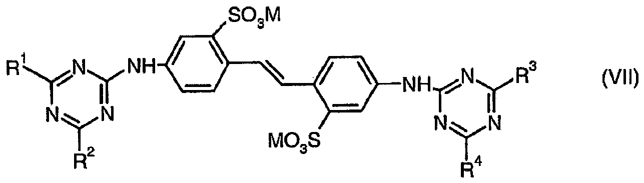 wherein
R¹ to R⁴ and M independently of the meaning in formula Ia, have the meaning that
R¹, R², R³, and R⁴ each, independently of one another, stand for OR⁵ or NR⁶R⁷, wherein
R⁵, R⁶, and R⁷ each, independently of one another, stand for hydrogen, substituted or unsubstituted alkyl, or substituted or unsubstituted aryl, wherein
R⁶, R⁷ can also form an aliphatic or aromatic ring together with the N atom to which they are bonded and optionally additional heteroatoms, and
M has the meaning according to claim 1 or 2,
is used as brightener.

8. The use according to claim 7, **characterized in that** in the compound VII at least one of the groups R¹ or R² has the same meaning as at least one of the groups R³ or R4.

9. The use according to claim 7 or 8, **characterized in that** in the compound VII the groups R¹, R², R³, and R4 independently of one another, stand for phenoxy, mono- or disulfonated phenoxy, phenylamino, mono- or disulfonated phenylamino, phenylamino substituted with C₁-C₃ alkyl, cyano, halogen, COOR, CONH-R, NH-COR, SO₂NH-R, or OR, also the groups morpholino, piperidino, pyrrolidino, -OC₁-C₄ alkyl, -NH-(C₁-C₄ alkyl), -N(C₁-C₄ alkyl)₂, -NH(C₂-C₄ alkylene)-OR, N[(C₂-C₄-alkylene)-OR]₂, -NH(C₂-C₄ hydroxylalkyl), -N(C₂-C₄ hydroxyalkyl)₂, -NH(C₂-C₄ alkylene-O-C₂-C₄-alkylene-OR), an amino acid or amino acid salt or an amino-acid amide, from whose amino group a hydrogen atom is removed, -N(CH₃)CH₂CH₂OH), -NH₂, -OCH₂CH₂SO₃M, -NH-CH₂CH₂SO₃M, -N(CH₂CH₂SO₃M)₂ or -N(CH₂CH₂OH)CH₂CH₂CONH₂, wherein R = H or C₁-C₃ alkyl and M has the meaning as defined above.

10. The use according to at least one of claims 7 to 9, **characterized in that** in the compound VII the groups R¹ to R⁴, independently of one another stand for -NH₂, -NH-CH₃, -NH-C₂H₅, -N(CH₃)₂, -N(C₂H₅)₂, -NH-C₂-C₄ hydroxyalkyl, -NH-CH₂CH₂SO₃M, -NH-CH₂-CH₂-O-CH₂-CH₂-OH, -OCH₃, -OCH(CH₃)₂, -O-CH₂-CH₂-O-CH₃, -N(CH₂-CH₂-OH)₂, -N(CH₂-CHOH-CH₃)₂, morpholino, -N(CH₂-CH₂-OH)CH₂-CH₂-CONH₂, as well as groups of the formula 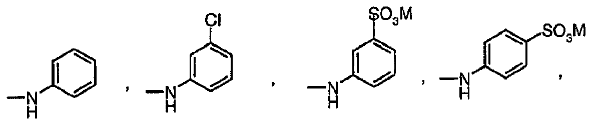 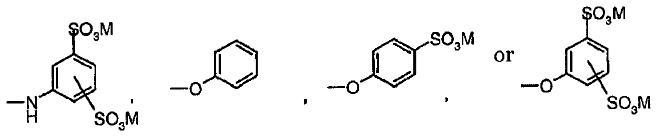 wherein M has the meaning as defined above.

11. The use according to at least one of claims 7 to 10, **characterized in that** in the compound VII the groups R¹ to R⁴, independently of each other, stand for 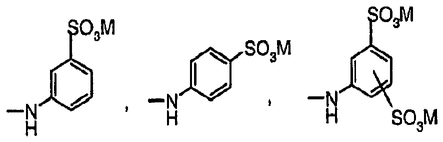 -NH-CH₂CH₂OH, -N(CH₂CH₂-OH)₂, N(CH₂-CHOH-CH₃)₂, anilino, or morpholino.

12. The use according to at least one of claims 1 to 3, **characterized in that** the compound of formula Ia is contained in a coating composition containing
- water,
- at least one white pigment,
- at least one binder, and
- at least one brightener as defined in claim 1 or a preparation as defined in at least one of claims 5 to 11.

13. The use according to claim 12, **characterized in that** the binder is a latex binder.

14. A coating composition containing
- water,
- at least one white pigment,
- at least one binder, and
- at least one brightener as defined in claim 1 or a preparation as defined in at least one of claims 5 to 11.

15. The coating composition according to claim 14, **characterized in that** the binder is a latex binder.

## Revendications

1. Utilisation d'un composé de formule Ia 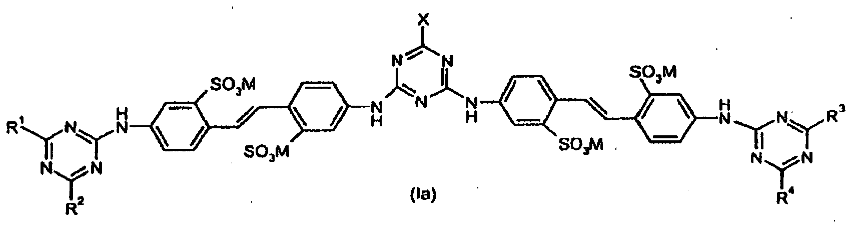 dans laquelle
R¹ et R³ représentent chacun indépendamment de l'autre un radical de formule 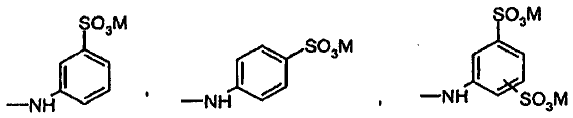
dans laquelle
M représente un atome d'hydrogène, un équivalent d'un ion métallique mono- ou divalent ou un ion ammonium éventuellement à substitution organique,
R² et R⁴ représentent chacun indépendamment de l'autre -NHCH₂-CH₂OH, -N(CH₂CH₂OH)₂, 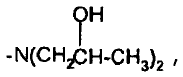 un groupe anilino ou morpholino, et
X représente -OH ou -NHCH₂CH₂OH, -N(CH₂CH₂OH)₂, 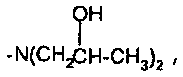 un groupe anilino ou morpholino,
en tant qu'azurant pour sauce de couchage du papier.

2. Utilisation selon la revendication 1, **caractérisée en ce que** M représente un équivalent d'un ion métallique mono- ou divalent du groupe des métaux alcalins ou alcalino-terreux.

3. Utilisation selon la revendication 1 ou 2, **caractérisée en ce que** R¹ = R³ et R² = R⁴.

4. Utilisation selon au moins l'une des revendication 1 à 3, **caractérisée en ce qu'**on prépare des composés de formule Ia, par le fait que l'on fait réagir un composé de formule II 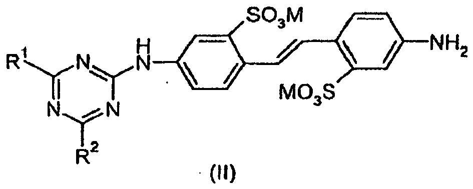 dans laquelle
R¹, R² et M ont les significations données au moins dans l'une des revendications 1 à 3, avec un composé de formule III
Y - B¹ -Z (III)
dans laquelle
Y et Z représentent chacun indépendamment de l'autre un groupe partant, qui peut être remplacé par le groupe amino libre des composés de formule II, et B¹ représente un radical de formule 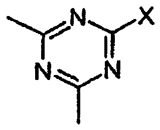
et X a les significations données ci-dessus,
pour obtenir un composé de formule IIIa 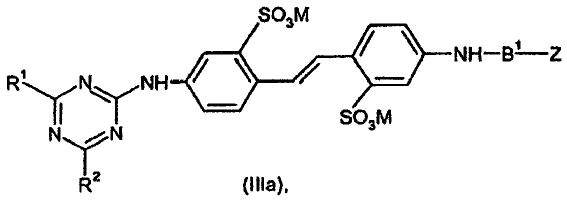 dans laquelle
R¹, R², B¹ et Z ont les significations données ci-dessus, et on fait encore réagir le composé IIIa avec un composé de formule IV 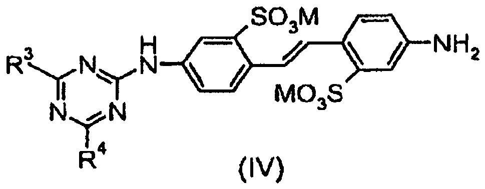
dans laquelle R³, R⁴ et M ont les significations données ci-dessus, pour obtenir le composé de formule Ia.

5. Utilisation selon au moins l'une des revendications 1 à 3, **caractérisée en ce que** le composé de formule I est contenu dans une préparation contenant, par rapport à la teneur totale en azurant, 1 à 15 % en aire d'au moins un composé selon l'une des revendications 1 à 3, le pourcentage en aire étant mesuré dans un chromatogramme en phase liquide haute pression pour une longueur d'onde de 350 nm sur une solution de mesure aqueuse de la préparation, la quantité de la préparation, exprimée en grammes, devant être utilisée comme prise d'essai dans 100 ml de la solution de mesure, multipliée par sa valeur E1/1, devant être égale à 50, la valeur E1/1 correspondant à la valeur fictive de l'extension, extrapolée à une solution à 1 % de l'échantillon à déterminer.

6. Utilisation selon la revendication 5, **caractérisée en ce que** la préparation contient de l'eau et 5 à 50 % en poids d'azurant total.

7. Utilisation selon au moins l'une des revendications 5 ou 6, **caractérisée en ce qu'**on utilise dans la préparation, en tant qu'azurant, un mélange du composé de formule la selon au moins l'une des revendications 1 à 3 et du composé VII 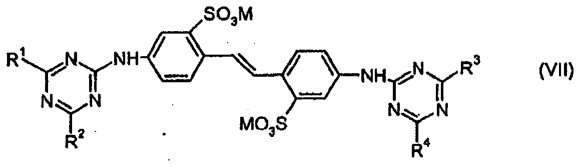 dans laquelle
R¹ à R⁴ et M ont, indépendamment de leurs définitions dans la formule Ia, des définitions selon lesquelles
R¹, R², R³ et R⁴ représentent chacun indépendamment des autres OR⁵ ou NR⁶R⁷, où
R⁵, R⁶ et R⁷ représentent chacun indépendamment des autres un atome d'hydrogène, un groupe alkyle substitué ou non substitué, ou aryle substitué ou non substitué, où
R⁶, R⁷, pris ensemble avec l'atome d'azote auquel ils sont liés et éventuellement à d'autres hétéroatomes, peuvent aussi former un noyau aliphatique ou aromatique, et
M a les significations selon la revendication 1 ou 2.

8. Utilisation selon la revendication 7, **caractérisée en ce que**, dans le composé VII, au moins l'un des radicaux R¹ ou R² a les mêmes significations qu'au moins l'un des radicaux R³ ou R⁴.

9. Utilisation selon la revendication 7 ou 8, **caractérisée en ce que**, dans le composé VII, les radicaux R¹, R², R³ et R⁴ représentent chacun indépendamment des autres un groupe phénoxy, phénoxy mono- ou disulfoné, phénylamino, phénylamino mono- ou disulfoné, phénylamino substitué par un ou des substituants alkyle en C₁-C₃n cyano, halogéno, -COOR, -CONH-R, -NH-COR, -SO₂NH-R ou -OR ; en outre un radical morpholino, pipéridino, pyrrolidino, -O(alkyle en C₁-C₄), -NH-(alkyle en C₁-C₄), -N(alkyle en C₁-C₄)₂, -NH(alkylène en C₂-C₄)-OR, -N[(alkylène en C₂-C₄)-OR]₂, -NH(hydroxyalkyle en C₂-C₄), -N(hydroxyalkyle en C₂-C₄)₂, -NH((alkylène en C₂-C₄)-O-(alkylène en C₂-C₄)-OR), un acide aminé ou un sel d'acide aminé ou un amide d'acide aminé, du groupe amino duquel a été éliminé un atome d'hydrogène, -N(CH₃) (CH₂CH₂OH), -NH₂, -OCH₂CH₂SO₃M, -NH-CH₂CH₂SO₃M, -N(CH₂CH₂SO₃M)₂ ou -N(CH₂CH₂OH) CH₂CH₂CONH₂, où R = H ou un groupe alkyle en C₁-C₃, et M a les significations données ci-dessus.

10. Utilisation selon au moins l'une des revendications 7 à 9, **caractérisée en ce que**, dans le composé VII les radicaux R¹ à R⁷ représentent chacun indépendamment des autres -NH₂ -NH-CH₃, -NH-C₂H₅, -N(CH₃)₂, -N(C₂H₅)₂, -NH-(hydroxyalkyle en C₂-C₄), -NH-CH₂CH₂SO₃M, -NH-CH₂-CH₂-O-CH₂-CH₂-OH, -OCH₃, -OCH(CH₃)₂, -O-CH₂-CH₂-O-CH₃, -N(CH₂-CH₂-OH)₂, -N(CH₂-CHOH-CH₃)₂, morpholino, -N(CH₂-CH₂-OH)CH₂-CH₂-CONH₂, ainsi qu'un radical de formule 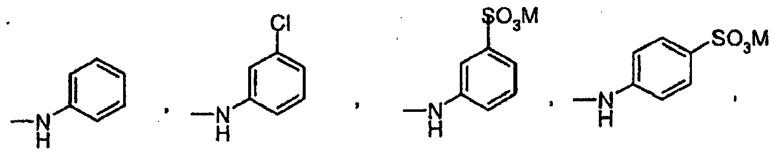 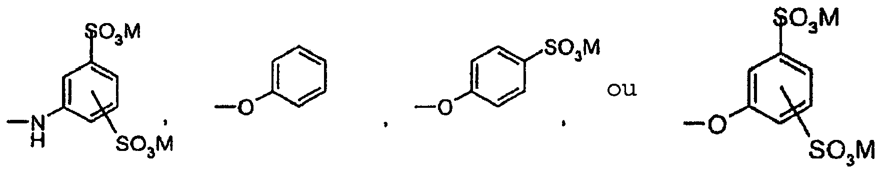 où M a les significations données ci-dessus.

11. Utilisation selon au moins l'une des revendications 7 à 10, **caractérisée en ce que**, dans le composé VII, les radicaux R¹ à R⁴ représentent chacun indépendamment des autres 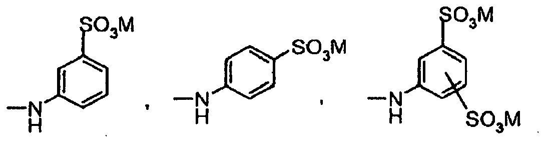 -NH-CH₂CH₂OH, N(CH₂-CH₂-OH)₂, -N(CH₂-CHOH-CH₃)₂, un groupe anilino ou morpholino.

12. Utilisation selon au moins l'une des revendications 1 à 3, **caractérisée en ce que** le composé de formule Ia est contenu dans une sauce de couchage contenant
- de l'eau,
- au moins un pigment blanc,
- au moins un liant, et
- au moins un azurant tel que défini dans la revendication 1 ou une préparation telle que définie dans au moins l'une des revendications 5 à 11.

13. Utilisation selon la revendication 12, **caractérisée en ce que** le liant est un liant latex.

14. Sauce de couchage, contenant
- de l'eau,
- au moins un pigment blanc,
- au moins un liant, et
- au moins un azurant tel que défini dans la revendication 1 ou une préparation telle que définie dans au moins l'une des revendications 5 à 11.

15. Sauce de couchage selon la revendication 14, **caractérisée en ce que** le liant est un liant latex.
